(19) Europäisches Patentamt
European Patent Office
Office européen des brevets

(11) **EP 2 441 793 B1**

(12) **EUROPÄISCHE PATENTSCHRIFT**

(45) Veröffentlichungstag und Bekanntmachung des Hinweises auf die Patenterteilung:
**15.08.2018 Patentblatt 2018/33**

(51) Int Cl.:
$C08J\ 5/18^{(2006.01)}$    $C08J\ 3/20^{(2006.01)}$
$C08K\ 5/00^{(2006.01)}$    $C08K\ 5/098^{(2006.01)}$
$C07C\ 51/41^{(2006.01)}$    $B29C\ 55/12^{(2006.01)}$
$B01D\ 67/00^{(2006.01)}$    $B01D\ 71/16^{(2006.01)}$
$H01M\ 2/16^{(2006.01)}$    $B01D\ 69/02^{(2006.01)}$
$B01D\ 71/26^{(2006.01)}$    $B29C\ 55/00^{(2006.01)}$
$C07C\ 55/02^{(2006.01)}$

(21) Anmeldenummer: **11002083.1**

(22) Anmeldetag: **14.03.2011**

(54) **HOCHAKTIVES BETA-NUKLEIERUNGSADDITIV FÜR POLYPROPYLEN**

HIGHLY ACTIVE BETA-NUCLEATION ADDITIVE FOR POLYPROPYLENE

ADDITIF DE NUCLÉATION BÉTA HAUTEMENT ACTIF POUR POLYPROPYLÈNES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2010 PCT/EP2010/006240**

(43) Veröffentlichungstag der Anmeldung:
**18.04.2012 Patentblatt 2012/16**

(73) Patentinhaber: **Treofan Germany GmbH & Co. KG
66539 Neunkirchen (DE)**

(72) Erfinder:
• **Busch, Detlef**
**66740 Saarlouis (DE)**
• **Klein, Dominic**
**66450 Bexbach (DE)**
• **Schmitz, Bertram**
**57200 Sarreguemines (FR)**

(74) Vertreter: **Kremer, Viola
Treofan Germany GmbH & Co. KG
Am Prime Parc 17
65479 Raunheim (DE)**

(56) Entgegenhaltungen:
WO-A1-02/081557    WO-A1-2011/047797
US-A- 3 240 552    US-A- 3 310 548

• MOHAMED M A ET AL: "A comparative study of the thermal reactivities of some transition metal oxalates in selected atmospheres", THERMOCHIMICA ACTA, ELSEVIER SCIENCE PUBLISHERS, AMSTERDAM, NL, Bd. 429, Nr. 1, 1. Mai 2005 (2005-05-01), Seiten 57-72, XP025386883, ISSN: 0040-6031 [gefunden am 2005-05-01]
• ZHAO S ET AL: "A highly active novel beta-nucleating agent for isotactic polypropylene", POLYMER, ELSEVIER SCIENCE PUBLISHERS B.V, GB, Bd. 49, Nr. 11, 27. Mai 2008 (2008-05-27), Seiten 2745-2754, XP022668139, ISSN: 0032-3861, DOI: 10.1016/J.POLYMER.2008.04.012 [gefunden am 2008-04-12]

EP 2 441 793 B1

**Beschreibung**

Die Erfindung betrifft ein Verfahren zur Erhöhung des Anteils der β-Kristallmodifikation in Polypropylen.

Von Polypropylen sind neben der amorphen Phase drei verschiedene kristalline, die α-, β-, und γ-Phasen bekannt. Beim Abkühlen von Polypropylenschmelzen bildet sich üblicherweise überwiegend das α-kristalline PP. Durch eine bestimmte Temperaturführung beim Abkühlen einer Polypropylenschmelze kann ein erhöhter Anteil an β-kristalliner Phase, erzeugt werden. Der auf diese Weise erzeugte Anteil an β-kristallinem PP beträgt weniger als 10%. Die hexagonale β-Modifikation des PP's zeichnet sich gegenüber der monoklinen α-Modifikation durch bessere mechanische Eigenschaften, insbesondere erhöhter Schlagzähigkeit und Spannungsrißbeständigkeit aus. Daneben weist die β-Modifikation des Polypropylens mit 148-155°C einen deutlich niedrigeren Schmelzpunkt gegenüber der α-Modifikation mit einem Schmelzpunkt von 160-170°C auf. Ein erhöhter Anteil an β-kristallinem Polypropylen wirkt sich daher in einigen Anwendungen günstig auf bestimmte Gebrauchseigenschaften des Polypropylens aus. Aus diesem Grund wurden in der Vergangenheit einige Additive entwickelt, die zu noch höheren Anteilen an Polypropylen in der β-Modifikation führen und daher im allgemeinen als β-Nukleatoren oder β-Nukleierungsmittel bezeichnet werden.

Als β-Nukleator mit hoher Aktivität ist der Farbstoff γ-Quinacridone in dem Deutschen Patent 1188278 beschrieben. Der Nachteil dieses Nukleierungsmittel ist jedoch die intensive Rotfärbung und die mangelnde thermische Stabilität, die oftmals beim Kompoundieren zur Zersetzung des Nukleierungsmittels und damit zum Verlust seiner Aktivität führt.

Im US-Patent 3540979 ist das Calciumsalz der Phtalsäure als thermisch stabiles β-Nukleierungsmittel beschrieben. Der Nachteil dieses Nukleierungsmittel ist die geringe Aktivität. Der damit erzielte Anteil an β-kristallinem Polypropylen beträgt höchsten 70% (K~0,5-0,7).

Ein zweikomponenten Nukleierungssystem aus Calciumcarbonat und organischen Dicarbonsäuren beschreibt DE 3610644. Dieses Nukleierungssystem zeigt in der Praxis jedoch eine schwankende Aktivität. Daher mangelt es an Reproduzierbarkeit. Der direkten Einsatz von Calciumsalze verschiedener Dicarbonsäuren ist im Patent DE 4420989 beschrieben. Die β-nukleierende Wirkung verschiedener Dicarboxamide, insbesondere N,N-Dicyclohexyl-2,6-Naphtalen-dicarboxamide beschreibt EP 0557721. Nachteil dieses Nukleators sind, die hohen Eduktkosten, sowie komplizierte Syntheseschritte bei der Herstellung.

Die Aufgabe der vorliegenden Erfindung bestand darin, ein Verfahren zur Erzeugung von β-kristallinem Polypropylen zur Verfügung zu stellen, sowie geeignete β-Nukleierungsmittel mit hoher β-Aktivität. Mittels des Verfahrens sollen hohe β-Anteile reproduzierbar und zuverlässig erreicht werden können. Das Verfahren soll einfach und effizient durchführbar sein. Die Modifizierung den β-Nukleierungsmitteln darf die üblichen wichtigen Gebrauchseigenschaften des Polypropylens nicht beeinträchtigen.

Diese Aufgabe wird gelöst durch ein Verfahren zur Herstellung von Polypropylen mit einem erhöhten Anteil an β-kristallinem Polypropylen, bei dem mindestens ein Nickel(II)-Dicarbonsäuresalz und mindestens ein Polypropylen gemischt werden und bei einer Temperatur von mindestens 150°C aufschmolzen und anschließend derart abgekühlt werden, daß die abgekühlte Polypropylenschmelze einen erhöhten Anteil von β-kristallinem Polypropylen aufweist.

Diese Aufgabe wird auch gelöst durch ein Verfahren zur Herstellung einer biaxial verstreckten Flachfolie, bei dem ein Nickel(II)-Dicarbonsäuresalz und Polypropylen gemischt und in einem Extruder bei einer Temperatur von mindestens 150°C aufgeschmolzen und die Schmelze durch eine Flachdüse extrudiert und die Schmelze derart zu einer Vorfolie abgekühlt wird, daß ein Anteil von mindestens 50% (gemessen nach DSC, 1.Aufheizkurve) β-kristallines Polypropylen entsteht, und bei dem die Vorfolie erwärmt und in Längsrichtung verstreckt und abkühlt, anschließend erneut erwärmt und in Querrichtung verstreckt wird, und wobei die Temperatur bei der Längsstreckung so gewählt wird, daß sich das β-kristalline Polypropylen der Vorfolie in die alpha Modifikation des Polypropylens umwandelt.

Diese Aufgabe wird auch gelöst durch eine Mischung oder Compound aus Polypropylen und Ni(II)-Dicarbonsäuresalz.

Diese Aufgabe wird auch gelöst durch die Verwendung von Ni(II)-Dicarbonsäuresalzen als β-Nukleierungsmittel in Polypropylen

Diese Aufgabe wird auch gelöst durch ein Verfahren zur Herstellung von Ni(II)-Dicarbonsäuresalzen bei dem eine wässrige Lösung einer Dicarbonsäure hergestellt wird und dieser wässrigen Lösung eine Ni(II)Salz-Lösung oder ein Ni(II)-Salz hinzugefügt wird nud das ausgefallene Ni(II)-Dicarbonsäuresalz abgetrennt und getrocknet wird.

Diese Aufgabe wird auch gelöst durch ein Verfahren zur Herstellung von nanoskaligen Ni(II)-Dicarbonsäuresalzen bei dem eine wässrige Lösung einer Dicarbonsäure hergestellt wird und dieser wässrigen Lösung eine Ni(II)Salz-Lösung oder ein Ni(II)-Salz hinzugefügt wird nud das ausgefallene Ni(II)-Dicarbonsäuresalz abgetrennt und getrocknet wird und das getrocknete Ni(II)-Dicarbonsäuresalz in einem nicht-wässrigen flüssigen Medium aufgeschlämmt wird und die Schlämme zur Zerkleinerung der aufgeschlämmten Ni(II)-Dicarbonsäuresalz bearbeitet wird.

Die abhängigen Unteransprüche geben bevorzugte Ausführungsformen der Erfindung an.

Die vorliegende Erfindung beruht auf der Entdeckung, daß Nickel(II)-Dicarbonsäuresalze beim Abkühlen einer Polypropylen-Schmelze, die mindestens ein Nickel(II)-Dicarbonsäuresalz enthält, zur Bildung eines hohen Anteils von β-kristallinem Polypropylen führen.

Nickel(II)dicarbonsäuresalze [$Ni^{2+}(^-OOC-(CH_2)_x-COO^-)$] sind Salze des zweiwertigen Nickels. Dicarbonsäuren sind gesättigte aliphatische Dicarbonsäuren, die der allgemeinen Formel $HOOC-(CH_2)_x-COOH$ entsprechen, worin X 2 bis 10 beträgt. Bevorzugte Dicarbonsäuren sind Pimelinsäure (1,6-Hexandisäure mit X=4); Adipinsäure (1,5-Pentandicarbonsäure mit X=3) und Suberinsäure (1,7-Heptandisäure mit X=5).

Die Synthese der Nickel(II)-Dicarbonsäuresalze erfolgt über die an sich bekannte Fällungsreaktion der aliphatischen Dicarbonsäuren, z.B. Pimelinsäure, Adipinsäure oder Suberinsäure, mit zweiwertigen Nickelsalzen, wie beispielsweise Chloriden, Carbonaten oder Hydroxiden, wie beispielsweise $NiCl_2$, Nickelcarbonat oder $Ni(OH)_2$ in wässriger Lösung. $Ni(OH)2$ ist bevorzugt, da die Bildung von Salzsäure oder CO2 als Nebenprodukt vermieden wird. Für die Umsetzung wird im allgemeinen eine wäßrige Lösung der aliphatischen Dicarbonsäure vorgelegt. Die aliphatische Dicarbonsäure wird in Wasser gegeben und unter Rühren erwärmt bis sich die aliphatische Dicarbonsäure gelöst hat, beispielsweise bei einer Temperatur von 70 bis 95°C, vorzugsweise 75 bis 90°C. Anschließend wird zu dieser Dicarbonsäure-Lösung unter weiterem Rühren das Ni(II)-Salz, vorzugsweise $Ni(OH)2$ direkt oder eine wässrige Ni(II)salz-Lösung, vorzugsweise eine $Ni(OH)2$-Lösung, hinzugefügt. Die Reaktanten werden hierbei im allgemeinen in stöchiometrischen Mengen eingesetzt. Dabei fällt das Ni(II)-Dicarbonsäuresalz als feiner Niederschlag aus. Dieser Niederschlag sedimentiert, wird abgetrennt und durch geeignete Verfahren getrocknet, z.B. in einem Trockenschrank, bei 100 - 120°C vorgetrocknet. Anschließend wird, beispielsweise unter Vakuum, z.B. in einem Vakuumtrockenschrank, bei ca. 150 bis 200°C der Restfeuchtegehalt des Ni(II)-Dicarbonsäuresalzes weiter gesenkt. Vorzugsweise beträgt der Wassergehalt des getrockneten Ni(II)-Dicarbonsäuresalzes 0 - 2 Gew. %, vorzugsweise >0 bis 1 Gew.-%. Auf diese Weise wird ein trockenes, pulverförmiges Ni(II)-Dicarbonsäuresalz erhalten. Vorzugsweise wird nach diesem Verfahren auch Ni(II)-Adipat, Ni(II)-Pimelat oder Ni(II)-Suberat hergestellt.

Für die Herstellung von nanoskaligen Nickel(II)-Dicarbonsäuresalzen mit einer Partikelgröße von unter 500nm sind auch die vorstehend beschriebenen Umsetzungen von Ni-(II)-Salzen mit der jeweiligen Dicarbonsäure im wässrigen Medium und anschließender Trocknung geeignet. Nanoskalige Nickel(II)-Dicarbonsäuresalze haben im allgemeinen eine Teilchengröße von 1 bis 500nm, vorzugsweise 5 bis 300nm, wobei gleichzeitig Teilchen oder Agglomerate mit einer Teilchengröße von >1 $\mu$m zu weniger als 3%, vorzugsweise >0 bis <1% enthalten sind. Somit liegt auch die mittlere Teilchengröße der nanoskaligen Nickel(II)-Dicarbonsäuresalze im besagten Bereich von 1 bis 500nm, vorzugsweise 5 bis 300nm. Entsprechend beträgt die Teilchengröße von nicht-nanoskaligen Ni(II)-Dicarbonsäuresalzen >500nm, vorzugsweise 700nm bis 5$\mu$m, insbesondere 800nm bis 3$\mu$m.

Zur Herstellung der nanoskaligen Nickel(II)-Dicarbonsäuresalzen wird das trockene pulverförmige Nickel(II)-Dicarbonsäuresalz nach Fällung und Trocknung in einem nicht-wässrigen flüssigen Medium aufgeschlämmt und z.B. durch Vermahlen der Schlämme, bearbeitet Im allgemeinen werden mindestens 5 bis 60 Gew.-%, vorzugsweise 10 bis 50 Gew.-% insbesondere 15 bis 40 Gew.-%, bezogen auf das Gewicht der nicht-wässrigen flüssigen Phase, des Ni(II)-Dicarbonsäuresalzes in der nicht-wässrigen flüssigen Phase aufgeschlämmt. Dieser Schlamm wird anschließend vermahlen. Zur Zerkleinerung dienen beispielsweise gängige Mörsermühlen, Ultraschall oder eine Kugelmühle oder andere gängige Nassmahl- oder Zerkleinerungsprozesse. Hierbei wird das Ni(II)-Dicarbonsäuresalz auf eine Partikelgröße von 1 bis 500nm, insbesondere 5 bis 300nm zerkleinert. Das nanoskalige Ni(II)-Dicarbonsäuresalz bildet nach der Zerkleinerung in der nicht-wässrigen flüssigen Phase eine stabile Dispersion in der Agglomerate von mehr als 1000nm nur in geringen Mengen oder gar nicht mehr vorliegen. Der Übergang zu der nanoskaligen dispergierten Phase zeigt sich auch darin, daß das aufgeschlämmte Ni(II)-Dicarbonsäuresalz vor der Mahlung zunächst direkt, z.B. innerhalb weniger Minuten, wieder sedimentiert, aber nach der Vermahlung eine stabile, milchige, trübe Dispersion bildet, in der sich die Teilchen nicht mehr absetzen. Diese Dispersion ist für eine übliche Zeitdauer bis zur Verarbeitung im wesentlichen stabil, z.B. für eine Dauer von mindestens einer oder auch mehreren Stunden. Gegebenenfalls kann die Dispersion zusätzlich filtriert werden, um Agglomerate abzutrennen, die nach der Mahlung noch vorhanden sind. Das Filtermedium wird so gewählt, daß alle Teilchen mit einer Größe von >1$\mu$m abgetrennt werden und die Dispersion anschließend frei von Teilchen dieser Größe ist oder zumindest davon weniger als 1% enthält. Nach diesen Verfahren können nanoskalige Ni(II)-Pimelate, Ni(II)-Adipate und Ni(II)-Suberate hergestellt werden.

Unter einem nicht-wässrigen flüssigen Medium oder einer nicht-wässrigen flüssigen Phase wird im Sinne der vorliegenden Erfindung eine organische Verbindung verstanden, die bei Raumtemperatur flüssig ist und deren Wassergehalt <1Gew.-% beträgt, beispielsweise Alkohole, niedere Alkane, Ketone und ähnliche Flüssigkeiten. Bevorzugt sind Hexan, Heptan oder Ethanol, Butanol oder Isopropanol oder Aceton. Es können auch Mischungen dieser flüssigen Phasen verwendet werden

Trocknung bedeutet, je nach gegebenem Zusammenhang, im Sinne der vorliegenden Erfindung sowohl die Entfernung von Wasser oder Feuchte als auch die Abtrennung des nicht-wässrigen, flüssigen Mediums.

Die Dispersion kann entweder direkt mit dem Polypropylen, z.B. in Form von Pulver oder Granulat, vermischt, beispielsweise durch Auftrommeln, und getrocknet werden. Alternativ wird die nicht-wässrige flüssige Phase der Dispersion abgetrennt und das so erhaltene Pulver aus nanoskaligem Ni(II)-Dicarbonsäuresalz mit Polypropylen, in Form von Pulver oder Granulat, vermischt. Über beide mögliche Verfahrensvarianten erhält man eine Mischung aus nanoskaligen Ni(II)-Dicarbonsäuresalzen und Polypropylen. Die Abtrennung der nicht-wässrigen flüssigen Phase erfolgt in beiden Verfahren mit üblichen geeigneten Mitteln, beispielsweise durch Verdunsten, Abziehen im Vakuum, Abdestillieren oder mittels einer Filterpresse. Die Mischungen enthalten im allgemeinen 0 bis 2 Gew.-% der flüssigen Phase, vorzugsweise >0 bis 1 Gew.-%.

Alle vorstehend beschriebenen Nickel(II)-Dicarbonsäuresalze werden nach der Trocknung mit üblichen Verfahren mit dem Polypropylen vermischt oder compoundiert. Hierzu werden beispielsweise mechanische Vormischungen aus Polypropylengranulat und Nickel(II)-Dicarbonsäuresalz, gegebenenfalls als Dispersion, hergestellt. Vorzugweise wird aus Nickel(II)-Dicarbonsäuresalz und Polypropylen ein Compound hergestellt. Unter einem Compound wird im Sinne der vorliegenden Erfindung eine homogene Mischung aus mindestens einem Polypropylen und Ni(II)-Dicarbonsäuresalz als Additiv verstanden. Die Herstellung des Compounds erfolgt in üblicher Weise in dem die Vormischung oder Polypropylen und Ni(II)-Dicarbonsäuresalz bei geeigneten Temperaturen aufgeschmolzen wird, beispielsweise in einem Bereich von 160 bis 300°C. Das Aufschmelzen erfolgt vorzugsweise in einem geeigneten Extruder, beispielsweise in einem Zweischneckenextruder, welcher gleichzeitig eine gute Mischung des Ni(II)-Dicarbonsäuresalzes im Polypropylen gewährleistet. Die aufgeschmolzene Mischung wird zu Granulatkörnern extrudiert und diese bei geeigneten Temperaturen abgekühlt. Bei der Compoundierung können zusätzlich zu dem Polypropylen weitere Zusatzstoffe und/oder andere Polyolefine hinzugefügt werden, beispielsweise Polyethylene.

Die Mischung oder das Compound aus Polypropylen und Nickel(II)-Dicarbonsäuresalz enthält im allgemeinen mindestens 85 Gew.-%, vorzugsweise 95 bis <100 Gew.-%, insbesondere 97 bis <100 Gew.-%, eines Polypropylens, sowie gegebenenfalls weitere Zusatzstoffe und 0,0001 bis 5 Gew.-%, vorzugsweise 0,001 bis 3 Gew.-% Nickel(II)-Dicarbonsäuresalz. Insbesondere für Folienanwendungen ist ein Gehalt von 0,001 bis 2 Gew.-% Dicarbonsäuresalze in dem Compound oder der Mischung bevorzugt. Die Angaben in Gew.-% beziehen sich jeweils auf das Gewicht der Mischung oder des Compounds. Gegebenenfalls können auch verschiedene Nickel(II)-Dicarbonsäuresalz gemischt werden. Des weiteren können die Nickel(II)-Dicarbonsäuresalz auch zusammen mit weiteren an sich bekannten β-Nukleierungsmitteln eingesetzt werden.

Gegebenenfalls kann zusätzlich zur Vermeidung von Agglomeration, insbesondere bei den nanoskaligen Ni(II)-Dicarbonsäuresalzen, und zur Verbesserung der Dispergierbarkeit der Ni(II)-Dicarbonsäuresalze in der Polypropylen-Matrix eine oberflächenaktive Substanz, wie z.B. höherwertige Carbonsäuren, Silane, Amine oder Sulfonate zugesetzt werden. Besonders bevorzugt sind für diese Zwecke langkettige Fettsäuren, wie Ölsäure oder Stearinsäure. Der Zusatz dieser Hilfsstoffe erfolgt beispielsweise beim Vermischen der Ni(II)-Dicarbonsäuresalze mit Polypropylen oder bei der Herstellung der Dispersion oder beim Vermischen der Dispersion mit Polypropylen. Derartige Verfahren sind an sich im Stand der Technik bekannt, beispielsweise in Macromol. Mater. Eng. 275, 8-17 (2000) sowie in GAK 5/1988 Jahrgang 41, Seite 211 ff oder Macromol. Rapid Commun, 2001, 22, 176-180 beschrieben. Derartige Beschichtungen werden bevorzugte für nanoskalige Nickel(II)-Dicarbonsäuresalze verwendet.

Die erfindungsgemäßen Mischungen oder Compounds aus Polypropylen und Ni(II)-Dicarbonsäuresalz können direkt zur Herstellung von Produkten beispielsweise Spritzgußteilen, Folien, poröse Folien, Fasern etc. eingesetzt oder zusammen mit weiteren Polyolefinen und/oder weiteren Zusatzstoffen verarbeitet werden.

Polypropylene umfassen im Sinne der vorliegenden Erfindung Propylenhomopolymere und Propylenmischpolymerisate mit einem oder mehreren olefinischen Comonomeren mit 2 oder 4 bis 10 C-Atomen. Im allgemeinen enthalten die Propylenpolymer 70 bis 100 Gew.-%, vorzugsweise 80 bis 99 Gew.-%, insbesondere 90 bis 98 Gew.-%, Propylen. Der entsprechende Comonomergehalt von höchstens 30 Gew.-% bzw. 1 bis 20 Gew.-% bzw. 2 bis 10 Gew.-% besteht, wenn vorhanden, im allgemeinen aus Ethylen und/oder Butylen. Die Angaben in Gew.-% beziehen sich jeweils auf das Propylenpolymere. Geeignete Mischpolymerisate, welche vorzugweise Ethylen und/oder Butylen als Comonomer enthalten, sind statistische Mischpolymerisate oder Blockcopolymere. Bevorzugt sind auch isotaktische Propylenhomopolymere mit einem Schmelzpunkt von 140 bis 170°C, vorzugsweise von 155 bis 165°C, und einen Schmelzflußindex (Messung DIN 53 735 bei 21,6 N Belastung und 230°C) von 1,0 bis 50 g/10 min, vorzugsweise von 1,5 bis 20 g/10 min.

Der n-heptanlösliche Anteil der Polypropylene beträgt im allgemeinen 1 bis 10 Gew.-%, vorzugsweise 2-5 Gew.-% bezogen auf das Ausgangspolymere. Die Molekulargewichtsverteilung der Polypropylene kann variieren. Das Verhältnis des Gewichtsmittels $M_w$ zum Zahlenmittel $M_n$ liegt im allgemeinen zwischen 1 und 15, vorzugsweise bei 2 bis 10, ganz besonders bevorzugt bei 2 bis 6. Eine derartig enge Molekulargewichtsverteilung der Polypropylene erreicht man beispielsweise durch dessen peroxidischen Abbau oder durch Herstellung des Polypropylens mittels geeigneter Metallocenkatalysatoren.

In einer weiteren Ausführungsform der Erfindung ist das eingesetzte Polypropylen, insbesondere Propylenhomopolymere hochisotaktisch. Für derartige hochisotaktische Polypropylene beträgt der mittels $^{13}$C-NMR-Spektroskopie bestimmte Kettenisotaxie-Index (Triade) des n-heptanunlöslichen Anteils des Polypropylens mindestens 95 %, vorzugsweise 96 bis 99 %.

Nach dem erfindungsgemäßen Verfahren zur Herstellung von Polypropylen mit einem erhöhten Anteil an β-kristallinem Polypropylen wird zunächst eine Mischung oder ein Compound aus Polypropylen und Nickel(II)-Dicarbonsäuresalz bei geeigneten Temperaturen aufgeschmolzen. Das Aufschmelzen erfolgt vorzugsweise in einem geeigneten Extruder, beispielsweise in einem Zweischneckenextruder, welcher gleichzeitig eine gute Verteilung der Nickel(II)-Dicarbonsäuresalze im Polypropylen gewährleistet. Die Extrudertemperatur liegt dabei im allgemeinen in einem Bereich von 180 bis 280°C. Die aufgeschmolzene Mischung wird extrudiert und bei geeigneten Temperaturen abgekühlt. In anderen Verfahrensvarianten erfolgt die Herstellung der Mischung bzw. des Compounds wie vorstehend beschrieben in einem vorgelagerten Arbeitsschritt. Diese Mischungen oder Compounds werden anschließend in dem erfindungsgemäßen Verfahren zusammen mit einem weiteren Polypropylen eingesetzt, welches das gleiche Polypropylen wie in der Mischung/Compound oder ein davon verschiedenes Polypropylen sein kann. Die Mischungen oder Compounds können in einem beliebigen Extrusionswerkzeug oder in einem Kneter aufgeschmolzen und mit dem weiteren Polypropylen gemischt werden.

Es ist erfindungswesentlich, daß nach der Extrusion die Abkühlung der salzhaltigen Polypropylen-Schmelze derart erfolgt, daß die β-nukleierende Wirkung der Nickel(II)-Dicarbonsäuresalze zum Tragen kommt. Hierfür ist es bevorzugt die Schmelze langsam bei einer Temperatur in einem Bereich von 60 bis 140 °C, vorzugsweise bei 80 bis 130°C abzukühlen. Je näher diese Temperatur in der Nähe der Kristallisationstemperatur des β-kristallinen Polypropylens (<140°C) liegt, umso günstiger sind die Bedingungen für die Ausbildung der β-kristallinen Modifikation. Auf diese Weise kann über die Auswahl der Temperatur beim Abkühlen ein mehr oder weniger hoher Anteil an β-Polypropylen erzeugt werden. Zusätzlich hat die Verweildauer der abkühlenden Schmelze bei der jeweiligen Temperatur einen Einfluß auf den erzielten β-Anteil. Zur Erzielung eines größtmöglichen β-Anteils sollte die Schmelze langsam bei höheren Temperaturen (120-140°C) abgekühlt werden, wobei die notwendige Verweildauer bei der gegebenen Temperatur im Einzelfall auch von der Formgebung bei der Extrusion abhängt.

Je nach Anwendungsfall können auch niedrigere β-Anteile, wie beispielsweise 10 bis 50%, im Polypropylen ausreichend sein. Die β-nukleierenden Nickel(II)-Dicarbonsäuresalze wirken sich in diesen Fällen positiv aus, da die Abkühlrate erhöht werden kann, d.h. schnellere Abzugsgeschwindigkeiten eingesetzt werden können.

Mittels des erfindungsgemäßen Verfahrens ist es möglich bei entsprechenden Abkühlbedingungen einen Gehalt an β-Polypropylen von >50 bis <100 Gew.-%, vorzugsweise 70 bis 99 Gew.-%, insbesondere 90 bis 99% (DSC-Methode, 1.Aufheizkurve) zu erzielen. Beispielsweise wurden über DSC-Messungen (1.Aufheizkurve, Methode nachstehend beschrieben) an isotaktischem Propylenhomopolymer mit 0,2 Gew.-% Nickel(II)-Dicarbonsäuresalz ein Anteil von β-kristallinem Polypropylen von bis zu 98,5 % bestimmt.

Das erfindungsgemäße Verfahren kann vorteilhaft bei der Herstellung von Folien, Formkörpern, insbesondere Rohren und Schläuchen, Fasern und anderen Extrusionsprozeße angewendet werden. Der erhöhte β-Anteil im Polypropylen wirkt sich bei den verschiedensten Extrusionsanwendungen günstig aus, beispielsweise da die Extrusionstemperaturen reduziert werden können. Für einige Anwendungen ist ein erhöhter Anteil an β-kristallinem Polypropylen vorteilhaft, da hierdurch Gebrauchseigenschaften des Polypropylens verbessert werden, z.B. erreicht man eine höhere Kerbschlagzähigkeit und Spannungsrißbeständigkeit des Polypropylens. In einer weiteren Anwendung nutzt man den hohen β-Anteil im Polypropylen zur Herstellung von porösen Folien durch Umwandlung der β-Modifikation in die alpha-Modifikation bei der Verstreckung von Folien oder zur Erzeugung von rauhen Oberflächen einer verstreckten Folie aus.

Grundsätzlich können in dem erfindungsgemäßen Verfahren alle vorstehend beschriebenen Polypropylene, d.h. Propylenehomopolymere und/oder Propylencopolymere und/oder Propylenterpolymere, verwendet werden. Im allgemeinen ist es bevorzugt Propylenhomopolymere einzusetzen.

Es wurde gefunden, daß die Nickel(II)-Dicarbonsäuresalze auch sehr vorteilhaft in einem Verfahren zur Herstellung einer porösen, vorzugsweise biaxialen verstreckten, Folie oder auch einer Folie mit einer oder mehreren porösen Schichten eingesetzt werden können. Die hohen Gehalte an β-Polypropylen wirken sich positiv auf die Porosität dieser Folie, bzw. der porösen Schicht, und deren Gasdurchlässigkeit aus. Bei Verwendung der erfindungsgemäßen Nickel(II)-Dicarbonsäuresalze können Streckbedingungen, insbesondere hohe Streckfaktoren angewendete werden, die zu einer besonders hohen Porosität der Folie, bzw. der Schicht führen, wobei gleichzeitig eine überraschend gute Laufsicherheit der Folie gegeben ist. Gegebenenfalls kann die Erfindung auch bei einer mehrschichtigen Folie genutzt werden, die neben einer oder mehreren porösen Schicht/en auch eine weitere oder mehrere im wesentlichen gasundurchlässige Schicht/en, beispielsweise aus Polyolefinen umfaßt. Die Angaben in dieser Beschreibung bezüglich der porösen Folie gelten somit sinngemäß in gleicher Weise oder analog auch für die poröse Schicht oder die porösen Schichten einer mehrschichtigen Folie.

Im Einzelnen werden bei der Herstellung einer biaxial verstreckten porösen Polypropylenfolie alle Komponenten der porösen Schicht oder Schichten in einem oder mehreren Extruder bei einer Temperatur von mindestens 160°C aufgeschmolzen. Die einoder mehrschichtige Polymerschmelze wird durch eine Flachdüse co/extrudiert, von einer Abnahmewalze aufgenommen und auf der Abnahmewalze derart abgekühlt, daß sich die Schmelze zu einer Vorfolie verfestigt und der gewünschte Anteil an β-kristallinem Polypropylen entsteht. Diese Abkühlung der Schmelze erfolgt vorzugsweise in einem Temperaturbereich von 80 bis 130°C, wobei eine lange Verweildauer bei dieser Temperatur zu einem erhöhten β-Polypropylen-Anteil beiträgt. Für die Herstellung einer porösen Folien, bzw. Schicht wird im allgemeinen ein Anteil von mindestens 40%, vorzugsweise 60 bis 98% an β-Polypropylen in der Vorfolie (gemessen nach DSC, 1.Aufheizen) angestrebt, wohingegen zur Erzeugung von Oberflächenrauhigkeiten geringere Anteile von beispielsweise 10 bis 40% ausreichend sein können. Anschließend wird die Vorfolie in an sich bekannter Weise erwärmt und in Längsrichtung verstreckt, vorzugsweise bei einer Temperatur von weniger als 140°C, insbesondere 80 bis 125°C und mit einem Streckfaktor von 2,5:1 bis 6:1. Nach der Längsstreckung wird die längsgestreckte Folie erneut erwärmt und in Querrichtung verstreckt, vorzugsweise bei einer Temperatur größer 110°C, insbesondere von 120 bis 145°C und mit einem Streckverhältnis von 3:1 bis 8:1. Durch die gewählten Temperaturen bei der Verstreckung wandelt sich das β-kristalline Polypropylen der Vorfolie in die alpha Modifikation des Polypropylens um und erzeugt je nach Verfahrensbedingungen und β-Anteil in der Vorfolie eine durchgehende poröse netzwerkartige Struktur in der Folie, bzw. in der porösen Schicht oder zumindest eine Oberflächenrauhigkeit durch kraterartige Vertiefungen, die bei den Umwandlungsprozeßen entstehen. Derartige rauhe Oberflächenstrukturen sind beispielsweise bei Folien mit papierähnlichem Charakter oder bei Kondensatorfolien erwünscht, welche als Dielektrikum in Kondensatoren eingesetzt werden. Um die elektrischen Eigenschaften solcher Kondensatorfolien nicht zu beeinträchtigen ist es bevorzugt das Nickel(II)-Dicarbonsäuresalze nur in der oder den Deckschicht/en einzusetzen, welche die Oberflächenrauheit aufweisen sollen. Es wurde gefunden, daß die, insbesonderen, nano-skaligen Ni(II)-Dicarbonsäuresalze die elektrischen Eigenschaften der Kondensatorfolie nicht oder nur geringfügig beeinträchtigen.

Überraschenderweise hat die Folie, bzw. die Schicht, welche die erfindungsgemäßen Nickel(II)-Dicarbonsäuresalzen enthält, eine sehr hohe und gleichmäßige Porosität und eine gute mechanische Festigkeit. Die gleichmäßige Verteilung der Porengröße ist in REM-Aufnahmen gut zu erkennen. Der mittlere Porendurchmesser (Bubble Point) liegt im Bereich von 10 bis 350nm, bevorzugt im Bereich 40 bis 300nm. Bei der Herstellung der porösen Folie, bzw. der Folie mit poröser Schicht, kommt es nur sehr selten zu Abrissen, d.h. das Verfahren hat eine hohe Laufsicherheit. Die Folie kann mit sehr hohen Faktoren verstreckt werden, so daß außergewöhnlich hohe Porositäten erzielt werden können. Grundsätzlich kann der Gurley-Wert der verschiedenen Ausführungsformen der porösen Folie in einem breiten Bereich variieren. Für solche Folien, welche nur poröse Schichten umfassen und beispielsweise als Membran-Folien verwendet werden, liegt der Gurley Wert im allgemeinen in einem Bereich von 100 - 5000s; vorzugsweise 100 bis 2000s. Überraschenderweise lassen sich nach der vorliegenden Erfindung durch hohe Streckfaktoren auch poröse Folien mit sehr niedrigen Gurley Werten von 10 bis <100s, vorzugsweise 15 bis 80s, insbesondere 15 bis 50s noch verfahrenssicher herstellen. Auch lassen sich poröse Folien mit Gurley Werten <600s und Porositäten >50% mit einer Dicke unter 30 $\mu$m, vorzugsweise 10 - 25$\mu$m, insbesondere 12 - 20$\mu$m noch laufsicher herstellen.

Die porösen Folie, bzw. die poröse/n Schicht/en der Folie enthält/enthalten in einer weiteren Ausführungsform Nickel(II)-Dicarbonsäuresalzen und isotaktisches Propylenhomopolymer und Propylenblockcopolymer, sowie gegebenenfalls weitere Polyolefine, welche die Porosität nicht beeinträchtigen. In diesen Ausführungsformen enthält die Folie bzw. die poröse Schicht im allgemeinen 50 bis 85 Gew.-%, vorzugsweise 60 bis 75 Gew.-%, Propylenhomopolymere und 15 bis 50 Gew.-% Propylenblockcopolymere, vorzugsweise 25 bis 40 Gew.-%, und 0,001 bis 5 Gew.-%, vorzugsweise 50 - 10.000 ppm des Nickel(II)-Dicarbonsäuresalzes als β-Nukleierungsmittels, bezogen auf das Gewicht der porösen Schicht, bzw. bezogen auf das Gewicht der Folie. Gegebenenfalls sind zusätzlich übliche Additive in geringen Mengen von unter 2 Gew.-%, beispielsweise Stabilisatoren und Neutralisationsmittel enthalten. Für den Fall, daß weitere Polyolefine enthalten sind, wird der Anteil des Propylenhomopolymeren oder des Blockcopolymeren entsprechend reduziert. Polyolefin sind beispielsweise Polyethylene, wie HDPE oder MDPE. Im allgemeinen sind diese Polyethylene wie HDPE und MDPE mit dem Polypropylen unverträglich und bilden im Gemisch mit Polypropylen eine separate Phase, die sich in einer DSC Messung durch einen separaten Schmelzepeak im Bereich der Schmelztemperatur des Polyethylens zeigt. HDPE hat im allgemeinen Schmelzpunkt, gemessen mit DSC (Maximum der Schmelzkurve, Aufheizgeschwindigkeit 20 °C/min), liegt zwischen 120 und 145°C, vorzugsweise 125 - 140°C, einen MFI (50 N/190 °C) von größer 0,1 bis 50 g/10 min, vorzugsweise 0,6 bis 20 g/10min, gemessen nach DIN 53 735 und eine Kristallinität von 35 bis 80 %, vorzugsweise 50 bis 80 % und eine Dichte, gemessen bei 23 °C nach DIN 53 479, Verfahren A, oder ISO 1183, von >0,94 bis 0,97 g/cm3. MDPE hat im allgemeinen einen Schmelzpunkt, gemessen mit DSC (Maximum der Schmelzkurve, Aufheizgeschwindigkeit 20 °C/min), zwischen 115 und 130 °C, vorzugsweise 120 - 125°C, einen MFI (50 N/190 °C) von größer 0,1 bis 50 g/10 min, vorzugsweise 0,6 bis 20 g/10min, gemessen nach DIN 53 735, eine Dichte, gemessen bei 23 °C nach DIN 53 479, Verfahren A, oder ISO 1183, von >0,925 bis 0,94 g/cm$^3$.

Im allgemeinen wird die Menge der zusätzlichen Polymeren 0 bis <50 Gew.-%, vorzugsweise 0,5 bis 40 Gew.-%, insbesondere 1 bis 30 Gew.-% betragen, wenn diese zusätzlich enthalten sind. In diesen Fällen wird der vorstehend beschriebene Anteil an Polypropylenen oder Propylenblockcopolymeren entsprechend erniedrigt. In gleicher Weise gilt, daß der besagte Propylenpolymer- oder Propylenblockcopolymer-Anteil reduziert wird, wenn höhere Mengen von bis zu 2 Gew.-% Nukleierungsmittel eingesetzt werden.

Die poröse Folie kann einschichtig oder mehrschichtig sein. Die Dicke der porösen Folie liegt im allgemeinen in einem Bereich von 10 bis 200$\mu$m, vorzugsweise 15 bis 150$\mu$m, insbesondere 15 bis 100$\mu$m. Die Dichte der porösen Folie liegt im allgemeinen in einem Bereich von 0,1 bis 0,6 g/cm3, vorzugsweise 0,2 bis 0,5 g/cm3. Die poröse Folie kann mit einer Corona, Flamm- oder Plasmabehandlung versehen werden, um die Befüllung mit Elektrolyten zu verbessern. Gegebenenfalls kann die mikroporöse Folie eine Abschaltschicht umfassen, welche die Durchlässigkeit der Folie bei erhöhten Temperaturen herabsetzt.

Die porösen Folien können vorteilhaft als Membran verwendet werden, beispielsweise in Batterien, sekundären Batterien, in Superkondensatoren oder in ähnlichen Anwendungen.

Zur Charakterisierung der Rohstoffe und der Folien wurden die folgenden Meßmethoden benutzt:

Schmelzflußindex

Der Schmelzflußindex der Propylenpolymeren wurde nach DIN 53 735 bei 2,16 kg Belastung und 230 °C gemessen und bei 190°C und 2,16 kg für Polyethylene.

Schmelzpunkte

Bei der DSC-Messung wird dem Polymeren mit einer definierten Aufheizrate eine Wärmemenge pro Zeiteinheit zugeführt und der Wärmestrom gegen die Temperatur aufgetragen. Der Schmelzpunkt ist im Sinne der vorliegenden Erfindung das Maximum der DSC Kurve. Zur Bestimmung des Schmelzpunkts wird die DSC-Kurve mit einer Aufheiz- und Abkühlgeschwindigkeit von 10K/1min im Bereich von 20 bis 200°C aufgenommen. Für die Bestimmung des Schmelzpunkts der Polymeren wird wie üblich die zweite Aufheizkurve ausgewertet.

Dichte

Die Dichte $\rho$ wird nach DIN 53 479, Verfahren A, bestimmt.

Porosität

Die Porosität errechnet sich aus der an der porösen Folie bestimmten Dichte pF und der Dichte des Ausgangsrohstoffes Polyproylen wie folgt:

$$P \, [\%] = 100 \times (1 - \rho F) / \rho PP)$$

Dabei wurde für Polypropylen eine Dichte von 0,92g/cm$^3$ angenommen.

Permeabilität (Gurley-Wert)

Die Permeabilität der Folien wurde mit dem Gurley Tester 4110, nach ASTM D 726-58 gemessen. Dabei wird die Zeit (in sec) bestimmt die 100 cm$^3$ Luft benötigen, um durch eine Fläche von 1 Inch$^2$ (6,452 cm$^2$) zu permeieren. Die Druckdifferenz über der Folie entspricht dabei dem Druck einer Wassersäule von 12,4 cm Höhe. Die benötigte Zeit entspricht dann dem Gurley-Wert.

$\beta$-Gehalt

Der Anteil des $\beta$-kristallinen Polypropylens wird mittels DSC bestimmt. Die mit dem Nickel(II)-Dicarbonsäuresalz additivierte Probe (Mischung, Compound, Folie, Formkörper) wird in der DSC zunächst mit einer Aufheizrate von 10K/min auf 220°C erhitzt und aufgeschmolzen (1. Aufheizen). Danach wird sie mit einer Kühlrate von 10K/min auf 100°C abgekühlt, bevor sie mit einer Heizrate von 10K/min (2. Aufheizen) wieder aufgeschmolzen wird.

Aus der DSC Kurve des 1. Aufheizen wird aus dem Verhältnis der Schmelzenthalpien der $\beta$-kristallinen Phase (Hβ) zu der Summe der Schmelzenthalpien von $\beta$- und $\alpha$-kristalliner Phase (Hß + Ha) der Anteil an $\beta$-kristallinem Polypropylen der Probe bestimmt, d.h. der $\beta$-Anteil der in der vermessenen Probe vorliegt (unverstreckte Folie, Formkörper, Spritzgussteil). Der prozentuale Wert errechnet sich wie folgt:

$$Kß,DSC \, [\%] = 100 \times (Hß) / (Hß + H\alpha)$$

Aus der DSC Kurve des 2. Aufheizen wird aus dem Verhältnis der Schmelzenthalpien der $\beta$-kristallinen Phase (Hß) zu der Summe der Schmelzenthalpien von $\beta$- und $\alpha$-kristalliner Phase (Hß + Ha) der Kristallinitätsgrad Kβ,DSC (2. Aufheizen) bestimmt, der den $\beta$-Anteil der jeweiligen Polypropylenprobe angibt, der maximal erreicht werden kann.

Agglomerate und Teilchengröße

Die Teilchengröße der Nickel(II)-Dicarbonsäuresalze und das Vorliegen von Agglomeraten wird an Rasterelektronischen Mikroskop-Aufnahme (REM) der Proben bestimmt.

Zur Durchführung der REM-Aufnahmen an einer Folienprobe wird ein Zuschnitt von 5x5mm aus der biaxial verstreckten Folie ausgeschnitten und auf den Probenträger aufgeklebt. Anschließend wird in einer Sputtereinheit eine wenige Nanometer dicke Schicht eines Edelmetalls (Pt, Au, Pd..) auf die Oberfläche der Folie aufgebracht.

Die besputterte Probe wird dann über eine Schleuse in das REM eingebracht und dort im Hochvakuum mit einer Beschleunigungsspannung von mehreren kV abgescannt. Die Beschleunigungsspannung wird so gewählt, dass ein scharfes Bild entsteht, ohne daß sich die Folien-Matrix auf Grund thermischer Belastung deformiert. Die Teilchen sind in der Aufnahme so gut zu erkennen, daß die Größe der einzelnen Partikel mit Hilfe des Maßstabs ausgemessen werden kann.

Die entsprechende Bestimmung der Teilchengröße der Nickel(II)-Dicarbonsäuresalze im Compound erfolgt an einer Cast-Folie als Probekörper. Hierfür wird aus dem Compound eine ca. 120 bis 150$\mu$m unverstreckte Castfolie hergestellt. Mit dieser Cast-Folie wird verfahren wie oben beschrieben.

Die Folie, bzw. das Compound ist im Sinne der vorliegenden Erfindung frei von Agglomeraten, wenn in der REM-Aufnahme der Folien-Probe keine Teilchen mit einer Größe von mehr als einem 1$\mu$m gefunden werden oder wenn maximal ein Teilchen von >1$\mu$m vorhanden ist. Die mittlere Teilchengröße kann durch Ausmessen der Teilchengröße einer statisch ausreichenden Anzahl von Teilchen erfolgen. Entsprechend kann auch der Anteil an Agglomeraten von >1$\mu$m an Hand der REM Aufnahmen bestimmt werden.

Die Erfindung wird nun an Hand von Ausführungsbeispielen näher erläutert:

Beispiele 1: Herstellung von Nickelpimelat

Beispiel 1a:

Es wurde eine wäßrige Lösung mit 40g Pimelinsäure in 1000ml Wasser vorgelegt und auf 83°C erwärmt bis die Pimelinsäure vollständig gelöst war. Zu dieser Lösung wurde eine wäßrige Nickelhydroxid-Milch (23,2g Ni(OH)$_2$ in 200ml Wasser) unter Rühren hinzugefügt, wodurch Nickel(II)pimelat als weißer Niederschlag ausfiel. Der sedimentierte Niederschlag wurde abgenutscht und bei 130°C im Trockenschrank vorgetrocknet. Abschließend wurde bei 200°C für 24h in einem Vakuumtrockenschrank die Restfeuchte und Kristallwasser entfernt. Auf diese Weise wurde ein grobkörniges getrocknetes Pulver aus Nickel-Pimelat erhalten.

Beispiel 1b

100 g des getrockneten Nickel-Pimelats aus Beispiel 1a wurden in 500ml wasserfreiem (Wassergehalt <1Gew.-%) Isopropanol aufgeschlämmt und die Schlämme in eine Kugelmühle gegeben und vermahlen. Dabei entstand eine stabile milchartige Dispersion. REM Aufnahmen zeigen eine Teilchengröße der Teilchen in der Dispersion im Bereich von 75nm. Es wurden in den Proben keine Agglomerate mit einer Teilchengröße von mehr als 0,8$\mu$m gefunden.

Beispiel 1c:

Die milchartige Dispersion nach Beispiel 1b wurde unter Feuchtigkeitsausschluss bei 90°C für 10h im Ablufttrockner getrocknet. Es wurde ein feines weißes Pulver aus Nickel-Pimelat erhalten.

Beispiel 1d:

Es wurde ein Nickel(II)-Pimelat wie in Beispiel 1c beschrieben hergestellt. Im Unterschied zu Beispiel 1a wurde der Dispersion von Nickel(II)-Pimelat in Isopropanol 30Gew.-%, bezogen auf das Gewicht des Pimelats, Stearinsäure zugesetzt. Im übrigen wurde in gleicher Weise wie in Beispiel 1c beschrieben verfahren. Es wurde ein feines weißes Pulver aus Nickel-Pimelat erhalten.

Beispiel 2a

Das Pulver nach den Beispiel 1a wurde in einem Mischer mit Granulat aus isotaktischem Polypropylenhomopolymer mit einem Schmelzpunkt 162°C und einem MFI von 3g/10min (Exxon Escorene PP 4352 F1) vermischt. Diese Mischung wurde in einem Zweischneckenextruder aufgeschmolzen (Gehäusetemperatur von 240°C und 200 1/min$^{-1}$) und zu stäbchen-förmigen Körnern granuliert. Die Konzentration des Nickel-Pimelats betrug 0,2 Gew.-% bezogen auf das Polypropylen.

REM Aufnahmen an den Granulatkörnern (Probekörper Cast-Folie) zeigen feinverteiltes agglomeratfreies Nickelpimelat in der PP-Matrix. Mittels DSC-Analyse zeigt das Compound aus Polypropylen und Nickelpimelat einen β-Wert von 95.5% beim 2.Aufheizen.

Beispiel 2b

Die Dispersion nach den Beispiel 1b wurde in einem Mischer auf Granulat aus isotaktischem Polypropylenhomopolymer (Schmelzpunkt 162°C; MFI 3g/10min)aufgetrommelt. Die Konzentration des Nickel-Pimelats betrug 0,2 Gew.-% bezogen auf das Polypropylen. Das Granulat mit dem aufgetrommelten Nickelpimelat wurde wie in Beispiel 2a in einem Zweischneckenextruder aufgeschmolzen (Gehäusetemperatur von 240°C und 200 1/min$^{-1}$) und zu stäbchenförmigen Körnern granuliert.

REM Aufnahmen an den Granulatkörnern (Probekörper Cast-Folie) zeigen feinverteiltes agglomeratfreies Nickelpimelat in der PP-Matrix. Mittels DSC-Analyse zeigt das Compound aus Polypropylen und Nickelpimelat einen β-Wert von 98.5% beim 2.Aufheizen.

Beispiel 2c

Es wurde Beispiel 2a wiederholt. Anstelle des Nickelpimelat-Pulvers nach Beispiel 1a wurde daß Pulver hergestellt nach Beispiel 1c verwendet. Im übrigen wurde in gleicher Weise wie in Beispiel 2a beschrieben verfahren.

Auch diese REM Aufnahmen an den Granulatkörnern (Probekörper Cast-Folie) zeigen feinverteiltes agglomeratfreies Nickelpimelat in der PP-Matrix. Mittels DSC-Analyse zeigt das Compound aus Polypropylen und Nickelpimelat einen β-Wert von 98.5% beim 2.Aufheizen.

Beispiel 2d

Es wurde Beispiel 2a wiederholt. Anstelle des Nickelpimelat-Pulvers nach Beispiel 1a wurde daß Pulver hergestellt nach Beispiel 1d verwendet. Zusätzlich wurde die Konzentration des stearinsäurebeschichteten Nickelpimelats auf 0,04 Gew.-% gesenkt. Im übrigen wurde in gleicher Weise wie in Beispiel 2a beschrieben verfahren.

Auch diese REM Aufnahmen an den Granulatkörnern (Probekörper Cast-Folie) zeigen feinverteiltes agglomeratfreies Nickelpimelat in der PP-Matrix. Mittels DSC-Analyse zeigt das Compound aus Polypropylen und Nickelpimelat einen β-Wert von 98.5% beim 2.Aufheizen.

Beispiele 3: Herstellung von Nickeladipat

Beispiel 3a:

Es wurde eine wäßrige Lösung mit 36,5g Adipinsäure in 1000ml Wasser vorgelegt und auf 83°C erwärmt bis die Adipinsäure vollständig gelöst war. Zu dieser Lösung wurde eine wäßrige Nickelhydroxid-Milch (23,2g Ni(OH)$_2$ in 200ml Wasser) unter Rühren hinzugefügt, wodurch Nickel(II)adipat als weißer Niederschlag ausfiel. Der sedimentierte Niederschlag wurde abgenutscht und bei 130°C im Trockenschrank vorgetrocknet. Abschließend wurde bei 200°C für 24h in einem Vakuumtrockenschrank die Restfeuchte und Kristallwasser entfernt. Auf diese Weise wurde ein grobkörniges getrocknetes Pulver aus Nickel-Adipat erhalten.

Beispiel 3b

100 g des getrockneten Nickel-Adipats aus Beispiel 1a wurden in 500ml wasserfreiem (Wassergehalt <1Gew.-%) Isopropanol aufgeschlämmt und die Schlämme in eine Kugelmühle gegeben und vermahlen. Dabei entstand eine stabile milchartige Dispersion. REM Aufnahmen zeigen eine Teilchengröße der Teilchen in der Dispersion im Bereich von 60nm. Es wurden in den Proben keine Agglomerate mit einer Teilchengröße von mehr als 0,8μm gefunden.

Beispiel 3c:

Die milchartige Dispersion nach Beispiel 1b wurde unter Feuchtigkeitsausschluss bei 90°C für 10h im Ablufttrockner getrocknet. Es wurde ein feines weißes Pulver aus Nickel-Adipat erhalten.

Beispiel 3d:

Es wurde ein Nickel(II)-Adipat wie in Beispielen 1a bis 1c beschrieben hergestellt. Im Unterschied zu 1b wurde der Dispersion von Nickel(II)-Adipat in Isopropanol 30 Gew.-%, bezogen auf das Gewicht des Adipats, Stearinsäure zugesetzt. Im übrigen wurde in gleicher Weise, wie in den Schritten 1a bis 1c beschrieben, verfahren. Es wurde ein feines weißes Pulver aus Nickel-Adipat erhalten.

Beispiel 4a

Das Pulver nach Beispiel 3a wurde in einem Mischer mit Granulat aus isotaktischem Polypropylenhomopolymer mit einem Schmelzpunkt 162°C und einem MFI von 3g/10min (Exxon Escorene PP 4352 F1) vermischt. Diese Mischung wurde in einem Zweischneckenextruder aufgeschmolzen (Gehäusetemperatur von 240°C und 200 1/min$^{-1}$) und zu stäbchen-förmigen Körnern granuliert. Die Konzentration des Nickel-Adipats betrug 0,2 Gew.-% bezogen auf das Polypropylen.

REM Aufnahmen an den Granulatkörnern (Probekörper Cast-Folie) zeigen feinverteiltes agglomeratfreies Nickeladipat in der PP-Matrix. Mittels DSC-Analyse zeigt das Compound aus Polypropylen und Nickeladipat einen β-Wert von 90.0% beim 2.Aufheizen.

Beispiel 4b

Die Dispersion nach den Beispiel 3b wurde in einem Mischer auf Granulat aus isotaktischem Polypropylenhomopolymer (Schmelzpunkt 162°C; MFI 3g/10min)aufgetrommelt. Die Konzentration des Nickel-Adipats betrug 0,2 Gew.-% bezogen auf das Polypropylen. Das Granulat mit dem aufgetrommelten Nickeladipat wurde wie in Beispiel 3a in einem Zweischneckenextruder aufgeschmolzen (Gehäusetemperatur von 240°C und 200 1/min$^{-1}$) und zu stäbchen-förmigen Körnern granuliert.

REM Aufnahmen an den Granulatkörnern (Probekörper Cast-Folie) zeigen feinverteiltes agglomeratfreies Nickeladipat in der PP-Matrix. Mittels DSC-Analyse zeigt das Compound aus Polypropylen und Nickeladipat einen β-Wert von 92.5% beim 2.Aufheizen.

Beispiel 4c

Es wurde Beispiel 4a wiederholt. Anstelle des Nickeladipat-Pulvers nach Beispiel 3a wurde daß Pulver hergestellt nach Beispiel 3c verwendet. Im übrigen wurde in gleicher Weise wie in Beispiel 4a beschrieben verfahren.

Auch diese REM Aufnahmen an den Granulatkörnern (Probekörper Cast-Folie) zeigen feinverteiltes agglomeratfreies Nickeladipat in der PP-Matrix. Mittels DSC-Analyse zeigt das Compound aus Polypropylen und Nickeladipat einen β-Wert von 92.5% beim 2.Aufheizen.

Beispiel 4d

Es wurde Beispiel 4a wiederholt. Anstelle des Nickeladipat-Pulvers nach Beispiel 1a wurde daß Pulver hergestellt nach Beispiel 3d verwendet. Zusätzlich wurde die Konzentration des stearinsäurebeschichteten Nickeladipats auf 0,04 Gew.-% gesenkt. Im übrigen wurde in gleicher Weise wie in Beispiel 4a beschrieben verfahren.

Auch diese REM Aufnahmen an den Granulatkörnern (Probekörper Cast-Folie) zeigen feinverteiltes agglomeratfreies Nickeladipat in der PP-Matrix. Mittels DSC-Analyse zeigt das Compound aus Polypropylen und Nickeladipat einen β-Wert von 92.5% beim 2.Aufheizen.

Der jeweilige β-Anteil der Compounds nach den Beispielen 1a bis 1d sowie 3a bis 3d wurde wie beschrieben über DSC aus der 2. Aufheizkurve bestimmt.

| Beispiel Nr. | Nukleator | Konz. [Gew.:%] | max.β-Anteil (2.Aufheizkurve) [%] |
|---|---|---|---|
| 1a/2a | Nickel(II)-Pimelat (grobes Pulver) | 0,2 | 95,5 |
| 1b/2b | Nickel(II)-Pimelat (Dispersion) | 0,2 | 98,5 |
| 1c/2c | Nickel(II)-Pimelat (feines Pulver) | 0,2 | 98,5 |
| 1d/2d | Nickel(II)-Pimelat (feines Pulver aus Dispersion mit Stearinsäure) | 0,04 | 98,5 |

(fortgesetzt)

| Beispiel Nr. | Nukleator | Konz. [Gew.:%] | max.β-Anteil (2.Aufheizkurve) [%] |
|---|---|---|---|
| 3a/4a | Nickel(II)-Adipat (grobes Pulver) | 0,2 | 90,0 |
| 3b/4b | Nickel(II)-Adipat (Dispersion) | 0,2 | 92,5 |
| 3c/4c | Nickel(II)-Adipat (feines Pulver) | 0,2 | 92,5 |
| 3d/4d | Nickel(II)-Adipat (feines Pulver aus Dispersion mit Stearinsäure) | 0,04 | 92,5 |

Folienbeispiel 5c

In einem Mischer wurden 20 Gew.-% Compound aus Polypropylen und Nickelpimelat hergestellt nach Beispiel 2a mit 60 Gew.-% Propylenhomopolymer und 20 Gew.-% Propylenblockcopolymer, jeweils bezogen auf die Mischung, vermischt. Diese Mischung wurde in einem Extruder aufgeschmolzen und weiter homogenisiert. Nach dem Extrusionsverfahren wurde die Schmelze aus einer Breitschlitzdüse bei einer Extrusionstemperatur von 245 °C zu einer einschichtigen Folie extrudiert. Diese Folie hatte die folgende Zusammensetzung:

| ca. 80 Gew.-% | Propylenhomopolymerisat (PP) mit einem n-heptanlöslichen Anteil von 4,5 Gew.-% (bezogen auf 100% PP) und einem Schmelzpunkt von 165 °C; und einem Schmelzflußindex von 3,2 g/10 min bei 230 °C und 2,16 kg Belastung (DIN 53 735) und |
|---|---|
| ca. 20 Gew.-% | Propylen-Ethylen-Blockcopolymerisat mit einem Ethylenanteil von ca. 5 Gew.-% bezogen auf das Blockcopolymer und einem Schmelzflußindex (230°C und 2,16 kg) von 6 g/10min |
| 0,04 Gew.-% | Nickel- Pimelat als β-Nukleierungsmittel |

Die Folie enthielt zusätzlich Stabilisator und Neutralisationsmittel in üblichen Mengen.

Die Polymermischung wurde nach der Extrusion über eine erste Abzugswalze und ein weiteres Walzentrio abgezogen, abgekühlt und verfestigt, anschließend längsgestreckt, quergestreckt und fixiert, wobei im einzelnen die folgenden Bedingungen gewählt wurden:

| Extrusion: | Extrusionstemperatur 245 °C |
|---|---|
| Abkühlwalze: | Temperatur 125°C, |
| Abzugsgeschwindigkeit: | 1,5 m/min (Verweilzeit auf der Abzugswalze: 55 sec) |
| β-Gehalt der Vorfolie: | 67% |
| Längsstreckung: | Streckwalze T = 90 °C |
| Längsstreckung um den | Faktor 4 |
| Querstreckung: | Aufheizfelder T = 145 °C |
| Streckfelder | T = 145 °C |
| Querstreckung um den | Faktor 4 |

Die so hergestellte poröse Folie war ca. 30 μm dick und wies eine Dichte von 0,30 g/cm$^3$ auf und zeigte ein gleichmäßiges weiß-opakes Aussehen. Die Porosität betrug 66% und der Gurley-Wert 200 s. Bei der Folienherstellung traten über mehrere Stunden keine Abrisse auf.

Folienbeispiel 6d

Es wurde eine Folie wie in Folienbeispiel 5c beschrieben hergestellt. Im Unterschied zu Folienbeispiel 5c wurden jetzt das Compound aus Polypropylen und Nickeladipat, hergestellt nach Beispiel 4d, eingesetzt. Die Zusammensetzung und die Verfahrensparameter wurden gegenüber Beispeiel 5c nicht geändert.

Die so hergestellte poröse Folie war ca. 30 μm dick und wies eine Dichte von 0,32 g/cm$^3$ auf und zeigte ein gleichmäßiges weiß-opakes Aussehen. Die Porosität betrug 63% und der Gurley-Wert 240 s. Bei der Folienherstellung traten über mehrere Stunden keine Abrisse auf.

**Patentansprüche**

1.  Verfahren zur Herstellung von Polypropylen mit einem Anteil an β-kristallinem Polypropylen, **dadurch gekennzeichnet, dass**

    • mindestens ein Nickel(II)-Dicarbonsäuresalz und mindestens ein Polypropylen gemischt werden und
    • das Nickel(II)dicarbonsäuresalz der allgemeinen Formel [Ni$^{2+}$($^-$OOC-(CH$_2$)$_x$-COO$^-$)] entspricht und die Dicarbonsäure eine gesättigte aliphatische Dicarbonsäure ist, die der allgemeinen Formel HOOC-(CH$_2$)$_x$-COOH entspricht, worin X 2 bis 10 beträgt, und
    • die Mischung bei einer Temperatur von mindestens 150°C aufgeschmolzen und
    • anschliessend derart abgekühlt wird, dass die abgekühlte Polypropylenschmelze einen Anteil von mehr als 50 bis 100 Gew.-% an β-kristallinem Polypropylen aufweist, wobei dieser β-kristalline Anteil mittels DSC Messung, wie in der Beschreibung unter der Überschrift β-Gehalt beschrieben, bestimmt wird.

2.  Verfahren zur Herstellung von Polypropylen mit einem Anteil an β-kristallinem Polypropylen, **dadurch gekennzeichnet, dass**

    • mindestens ein Nickel(II)-Dicarbonsäuresalz und mindestens ein Polypropylen gemischt werden und
    • das Nickel(II)dicarbonsäuresalz der allgemeinen Formel [Ni$^{2+}$($^-$OOC-(CH$_2$)$_x$-COO$^-$)] entspricht und die Dicarbonsäure eine gesättigte aliphatische Dicarbonsäure ist, die der allgemeinen Formel HOOC-(CH$_2$)$_x$-COOH entspricht, worin X 2 bis 10 beträgt, und
    • die Mischung bei einer Temperatur von mindestens 150°C aufgeschmolzen und
    • anschliessend derart abgekühlt werden, dass die abgekühlte Polypropylenschmelze einen Anteil von mehr 10 bis 50 Gew.-% an β-kristallinem Polypropylen aufweist, wobei dieser β-kristalline Anteil mittels DSC Messung, wie in der Beschreibung unter der Überschrift β-Gehalt beschrieben, bestimmt wird.

3.  Verfahren nach Anspruch 1, **dadurch gekennzeichnet dass** beim Abkühlen der Polypropylenschmelze ein β-Anteil von 70 bis <100 % erzeugt wird.

4.  Verfahren nach einem der Ansprüche 1 bis 3, **dadurch gekennzeichnet, dass** die Abkühlung der Polypropylenschmelze in einem Temperaturbereich von 100 - 140°C erfolgt.

5.  Verfahren nach einem der Ansprüche 1 bis 4 **dadurch gekennzeichnet, dass** das Polypropylen Propylenhomopolymer und/oder Propylencopolymer und/oder Propylenterpolymer ist.

6.  Verfahren nach einem der Ansprüche 1 bis 5, **dadurch gekennzeichnet, dass** das Polypropylen ein isotaktisches Polypropylen mit einem Schmelzpunkt im Bereich von 140 bis 170°C ist.

7.  Verfahren nach einem der Ansprüche 1 bis 6, **dadurch gekennzeichnet, dass** das Nickel(II)-Dicarbonsäuresalz eine mittlere Teilchengrösse von 1 bis 500nm, aufweist, wobei die mittlere Teilchengröße, wie in der Beschreibung unter der Überschrift Agglomerate und Teilchengröße beschrieben, an rasterelektronischen Mikroskop-Aufnahmen (REM) bestimmt wird.

8.  Verfahren nach einem der Ansprüche 1 bis 6, **dadurch gekennzeichnet, dass** das Nickel(II)-Dicarbonsäuresalz eine mittlere Teilchengrösse von mehr als 500nm bis 5μm aufweist, wobei die mittlere Teilchengröße, wie in der Beschreibung unter der Überschrift Agglomerate und Teilchengröße beschrieben, an rasterelektronischen Mikroskop-Aufnahmen (REM) bestimmt wird.

9.  Verfahren nach einem der Ansprüche 1 bis 8, **dadurch gekennzeichnet, dass** das Nickel(II)-Dicarbonsäuresalz ein Adipat oder Pimelat oder Suberat ist.

10. Verfahren nach einem der Ansprüche 1 bis 9, **dadurch gekennzeichnet, dass** das Nickel(II)-Dicarbonsäuresalz mit einer Oberflächenbeschichtung aus langkettigen Fettsäuren, vorzugsweise Ölsäure, Stearinsäure, Silanen, Aminen oder Sulfonaten versehen ist.

11. Verfahren nach einem der Ansprüche 1 bis 10, **dadurch gekennzeichnet, dass** man ein Compound aus Polypropylen und Nickel(II)-Dicarbonsäuresalz herstellt und dieses Compound mit Polypropylen mischt, aufschmilzt und abkühlt.

12. Verfahren zur Herstellung einer biaxial verstreckten Flachfolie, **dadurch gekennzeichnet, dass**

- man Nickel(II)-Dicarbonsäuresalz und Polypropylen mischt und
- in einem Extruder bei einer Temperatur von mindestens 150°C aufschmilzt und
- die Schmelze durch eine Flachdüse extrudiert und
- die Schmelze derart zu einer Vorfolie abkühlt, dass ein Anteil von mindestens 50% β-kristallines Polypropylen entsteht, wobei dieser β-kristalline Anteil mittels DSC Messung, wie in der Beschreibung unter der Überschrift β-Gehalt beschrieben, bestimmt wird und
- danach die Vorfolie erwärmt und in Längsrichtung und in Querrichtung so verstreckt wird, dass sich das β-kristalline Polypropylen der Vorfolie in die alpha Modifikation des Polypropylens umwandelt,
- wobei die Nickel(II)dicarbonsäuresalze der allgemeinen Formel $[Ni^{2+}(^-OOC-(CH_2)_x-COO^-)]$ entsprechen und die Dicarbonsäuren gesättigte aliphatische Dicarbonsäuren sind, die der allgemeinen Formel $HOOC-(CH_2)_x-COOH$ entsprechen, worin X 2 bis 10 beträgt,

13. Verfahren nach Anspruch 12, **dadurch gekennzeichnet, dass** die biaxial orientierte Folie porös ist.

14. Mischung oder Compound aus Polypropylen und Ni(II)-Dicarbonsäuresalz, **dadurch gekennzeichnet, dass** das Nickel(II)-Dicarbonsäuresalz eine mittlere Teilchengrösse von weniger als 500nm aufweist, wobei die mittlere Teilchengröße, wie in der Beschreibung unter der Überschrift Agglomerate und Teilchengröße beschrieben, an rasterelektronischen Mikroskop-Aufnahmen (REM) bestimmt wird und das Nickel(II)dicarbonsäuresalz der allgemeinen Formel $[Ni^{2+}(^-OOC-(CH_2)_x-COO^-)]$ entsprechen und die Dicarbonsäuren gesättigte aliphatische Dicarbonsäuren sind, die der allgemeinen Formel $HOOC-(CH_2)_x-COOH$ entsprechen, worin X 2 bis 10 beträgt,

15. Mischung oder Compound nach Anspruch 14, **dadurch gekennzeichnet, dass** das Polypropylen ein isotaktisches Propylenhomopolymer, ein Propylencopolymer, ein Propylenterpolymer oder eine Mischung aus einem oder mehreren dieser Polypropylene ist.

16. Mischung oder Compound nach Anspruch 14 oder 15, **dadurch gekennzeichnet, dass** das isotaktische Propylenhomopolymer eine Isotaktizität von mindestens 95% aufweist, wobei die Isotaktizität mittels $^{13}$C-NMR Spektroskopie (Triade) am n-heptan unlöslichen Anteil des isotaktischen Propylenhomopolymers gemessen wird.

17. Mischung oder Compound nach Anspruch 14 oder 15, **dadurch gekennzeichnet, dass** das isotaktische Propylenhomopolymer eine Isotaktizität von weniger als 95% aufweist, wobei die Isotaktizität mittels $^{13}$C-NMR Spektroskopie (Triade) am n-heptan unlöslichen Anteil des isotaktischen Propylenhomopolymers gemessen wird.

18. Mischung oder Compound nach einem der Ansprüche 14 bis 17, **dadurch gekennzeichnet, dass** die Mischung oder das Compound einen maximalen β-Anteil von >90% aufweisst, wobei dieser maximale β-Anteil mittels DSC Messung aus der zweiten Aufheizkurve, wie in der Beschreibung unter der Überschrift β-Gehalt beschrieben, bestimmt wird.

19. Mischung oder Compound nach einem der Ansprüch 14 bis 18, **dadurch gekennzeichnet, dass** das Ni(II)-Dicarbonsäuresalz eine Ni(II)-Pimelat, Ni(II)-Adipat oder Ni(II)-Suberat ist.

20. Mischung oder Compound nach einem der Ansprüch 14 bis 19, **dadurch gekennzeichnet, dass** die Ni(II)-Dicarbonsäuresalz mit langkettigen Fettsäuren, Silanen, Aminen oder Sulfonaten, vorzugsweise mit Ölsäure oder Stearinsäure beschichtet sind.

21. Verwendung von Ni(II)-Dicarbonsäuresalzen als β-Nukleierungsmittel in Polypropylen, wobei die Nickel(II)dicarbonsäuresalze der allgemeinen Formel $[Ni^{2+}(^-OOC-(CH_2)_x-COO^-)]$ entsprechen und die Dicarbonsäuren gesättigte aliphatische Dicarbonsäuren sind, die der allgemeinen Formel $HOOC-(CH_2)_x-COOH$ entsprechen, worin X 2 bis 10 beträgt,

22. Verfahren zur Herstellung von nanoskaligen Ni(II)-Dicarbonsäuresalzen, bei dem eine wässrige Lösung einer Dicarbonsäure hergestellt wird und dieser wässrigen Lösung eine Ni(II)-Salz-Lösung oder ein Ni(II)-Salz hinzugefügt wird und das ausgefallene Ni(II)-Dicarbonsäuresalz abgetrennt und getrocknet wird und das getrocknete Ni(II)-Dicarbonsäuresalz in einem nicht-wässrigen flüssigen Medium aufgeschlämmt wird und die Schlämme zur Zerkleinerung der aufgeschlämmten Ni(II)-Dicarbonsäuresalz bearbeitet wird, wobei die Nickel(II)dicarbonsäuresalze der allgemeinen Formel $[Ni^{2+}(^-OOC-(CH_2)_x-COO^-)]$ entsprechen und die Dicarbonsäuren gesättigte aliphatische Dicarbonsäuren sind, die der allgemeinen Formel HOOC-$(CH_2)_x$-COOH entsprechen, worin X 2 bis 10 beträgt,

23. Verfahren nach Anspruch 22, **dadurch gekennzeichnet, dass** das Ni(II)-Salz $NiCl_2$, Nickelcarbonat oder $Ni(OH)_2$ ist.

24. Folie, umfassend Polypropylen und Ni(II)-Dicarbonsäuresalz, wobei das Nickel(II)dicarbonsäuresalz der allgemeinen Formel $[Ni^{2+}(^-OOC-(CH_2)_x-COO^-)]$ entsprechen und die Dicarbonsäure eine gesättigte aliphatische Dicarbonsäure ist, die der allgemeinen Formel HOOC-$(CH_2)_x$-COOH entspricht, worin X 2 bis 10 beträgt,

25. Folie nach Anspruch 24, **dadurch gekennzeichnet, dass** das Nickel(II)-Dicarbonsäuresalz eine mittlere Teilchengrösse von weniger als 500nm aufweist, wobei die mittlere Teilchengröße, wie in der Beschreibung unter der Überschrift Agglomerate und Teilchengröße beschrieben, an rasterelektronischen Mikroskop-Aufnahmen (REM) bestimmt wird.

26. Folie nach Anspruch 24 oder 25, **dadurch gekennzeichnet, dass** das Nickel(II)-Dicarbonsäuresalz eine mittlere Teilchengrösse von mehr als 500nm, bis 5μm, aufweist, wobei die mittlere Teilchengröße, wie in der Beschreibung unter der Überschrift Agglomerate und Teilchengröße beschrieben, an rasterelektronischen Mikroskop-Aufnahmen (REM) bestimmt wird.

**Claims**

1. A method for producing polypropylene with a β-crystalline polypropylene content, **characterized in that**

   • at least one nickel(II) dicarboxylic acid salt and at least one polypropylene are mixed and
   • the nickel(II) dicarboxylic acid salt corresponds to the general formula $[Ni^{2+}(^-OOC-(CH_2)_x-COO^-)]$, and the dicarboxylic acid is a saturated aliphatic dicarboxylic acid which corresponds to the general formula HOOC-$(CH_2)_x$-COOH, where X is 2 to 10, and
   • the mixture is melted at a temperature of at least 150°C, and
   • then is cooled so that the cooled polypropylene melt contains an amount of more than 50% to 100% by weight β-crystalline polypropylene, wherein this β-crystalline content is determined by means of DSC measurement, as described under the heading "β content" in the specification.

2. The method for producing polypropylene with a β-crystalline polypropylene content, **characterized in that**

   • at least one nickel(II) dicarboxylic acid salt and at least one polypropylene are mixed and
   • the nickel(II) dicarboxylic acid salt corresponds to the general formula $[Ni^{2+}(^-OOC-(CH_2)_x-COO^-)]$, and the dicarboxylic acid is a saturated aliphatic dicarboxylic acid which corresponds to the general formula HOOC-$(CH_2)_x$-COOH, where X is 2 to 10 and
   • the mixture is melted at a temperature of at least 150°C and
   • then is cooled so that the cooled polypropylene melt contains an amount of more than 10% to 50% by weight β-crystalline polypropylene, wherein this β-crystalline content is determined by means of DSC measurement, as described under the heading "β content" in the specification.

3. The method according to claim 1, **characterized in that** a β content of 70 to <100% is created by cooling the polypropylene melt.

4. The method according to any one of claims 1 to 3, **characterized in that** the polypropylene melt is cooled in a temperature range of 100-140°C.

5. The method according to any one of claims 1 to 4, **characterized in that** the polypropylene is a propylene homopolymer and/or propylene copolymer and/or propylene terpolymer.

6. The method according to any one of claims 1 to 5, **characterized in that** the polypropylene is an isotactic polypropylene with a melting point in the range of 140-170°C.

7. The method according to any one of claims 1 to 6, **characterized in that** the nickel(II) dicarboxylic acid salt has an average particle size of 1 to 500 nm, wherein the average particle size is determined on scanning electron micrographs (SEM) as described under the heading "agglomerates and particle size" in the specification.

8. The method according to any one of claims 1 to 6, **characterized in that** the nickel(II) dicarboxylic acid salt has an average particle size of more than 500 nm up to 5 $\mu$m, wherein the average particle size is determined on scanning electron micrographs (SEM) as described under the heading "agglomerates and particle size" in the specification.

9. The method according to any one of claims 1 to 8, **characterized in that** the nickel(II) dicarboxylic acid salt is an adipate or pimelate or suberate.

10. The method according to any one of claims 1 to 9, **characterized in that** the nickel(II) dicarboxylic acid salt is provided with a surface coating of long-chain fatty acids, preferably oleic acid, stearic acid, silanes, amines or sulfonates.

11. The method according to any one of claims 1 to 9, **characterized in that** a compound of polypropylene and nickel(II) dicarboxylic acid salt is prepared, and this compound is mixed with polypropylene, melted and cooled.

12. The method for producing a biaxially drawn flat film, **characterized in that**

- nickel(II) dicarboxylic acid salt and polypropylene are mixed, and
- melted in an extruder at a temperature of at least 150°C, and
- the melt is extruded through a flat nozzle and
- the melt is cooled to form a pre-film such that an amount of at least 50% $\beta$-crystalline polypropylene is formed, wherein this $\beta$-crystalline content is determined by means of DSC measurement, as described under the heading "$\beta$ content" in the specification, and
- then the pre-film is heated and drawn in the longitudinal direction and in the transverse direction, so that the $\beta$-crystalline polypropylene of the pre-film is converted into the alpha modification of the polypropylene,
- wherein the nickel(II) dicarboxylic acid salts correspond to the general formula $[Ni^{2+}(^-OOC\text{-}(CH_2)_x\text{-}COO^-)]$, and the dicarboxylic acids are saturated aliphatic dicarboxylic acids corresponding to the general formula $HOOC\text{-}(CH_2)_x\text{-}COOH$, where X is 2 to 10.

13. The method according to claim 12, **characterized in that** the biaxially oriented film is porous.

14. A mixture or compound of polypropylene and nickel(II) dicarboxylic acid salt, **characterized in that** the nickel(II) dicarboxylic acid salt has an average particle size of less than 500 nm, wherein the average particle size, as described under the heading "agglomerates and particle size" in the specification, is determined on scanning electron micrographs (SEM), and the nickel(II) dicarboxylic acid salt corresponds to the general formula $[Ni^{2+}(^-OOC\text{-}(CH_2)_x\text{-}COO^-)]$ and the dicarboxylic acids are saturated aliphatic dicarboxylic acids, which correspond to the general formula $HOOC\text{-}(CH_2)_x\text{-}COOH$, where X is 2 to 10.

15. The mixture or compound according to claim 14, **characterized in that** the polypropylene is an isotactic propylene homopolymer, a propylene copolymer, a propylene terpolymer or a mixture of one or more of these polypropylenes.

16. The mixture or compound according to claim 14 or 15, **characterized in that** the isotactic propylene homopolymer has an isotacticity of at least 95%, wherein the isotacticity is measured by means of $^{13}$C-NMR spectroscopy (triads) on the fraction of the isotactic propylene homopolymer not soluble in n-heptane.

17. The mixture or compound according to claim 14 or 15, **characterized in that** the isotactic propylene homopolymer has an isotacticity of at least 95%, wherein the isotacticity is measured by means of $^{13}$C-NMR spectroscopy (triads) on the fraction of the isotactic propylene homopolymer not soluble in n-heptane [sic or tn].

18. The mixture or compound according to claims 14 to 17, **characterized in that** the mixture or the compound has a maximum $\beta$ content of >90%, wherein this maximum $\beta$ content is determined by means of DSC measurement from the second heating curve as described under the heading "$\beta$ content" in the specification.

19. The mixture or compound according to claims 14 to 18, **characterized in that** the mixture or the Ni(II) dicarboxylic acid salt is an Ni(II) pimelate, Ni(II) adipate or Ni(II) suberate.

20. The mixture or compound according to claims 14 to 19, **characterized in that** the mixture or the Ni(II) dicarboxylic acid salt is coated with long-chain fatty acids, silanes, amines or sulfonates, preferably with oleic acid or stearic acid.

**21.** Use of Ni(II) dicarboxylic acid salts as $\beta$-nucleating agents in polypropylene, wherein the nickel(II) dicarboxylic acid salts correspond to the general formula $[Ni^{2+}(^-OOC\text{-}(CH_2)_x\text{-}COO^-)]$ and the dicarboxylic acids are saturated aliphatic dicarboxylic acids which correspond to the general formula $HOOC\text{-}(CH_2)_x\text{-}COOH$, where X is 2 to 10.

**22.** A method for producing nanoscale Ni(II) dicarboxylic acid salts, wherein// in which an aqueous solution of a dicarboxylic acid is prepared and an Ni(II) salt solution or an Ni(II) salt is added to this aqueous solution, and the precipitated Ni(II) dicarboxylic acid salt is separated and dried, and the dried Ni(II) dicarboxylic acid salt is slurried in a nonaqueous liquid medium, and the slurries are processed to pulverize the slurried Ni(II) dicarboxylic acid salt, wherein the nickel(II) dicarboxylic acid salts correspond to the general formula $[Ni^{2+}(^-OOC\text{-}(CH_2)_x\text{-}COO^-)]$ and the dicarboxylic acids are saturated aliphatic dicarboxylic acids which correspond to the general formula $HOOC\text{-}(CH_2)_x\text{-}COOH$, where X is 2 to 10.

**23.** The method according to claim 22, **characterized in that** the Ni(II) salt is $NiCl_2$, nickel carbonate or $Ni(OH)_2$.

**24.** A film comprising polypropylene and Ni(II) dicarboxylic acid salt, wherein the nickel(II) dicarboxylic acid salt corresponds to the general formula $[Ni^{2+}(^-OOC\text{-}(CH_2)_x\text{-}COO^-)]$, and the dicarboxylic acid is a saturated aliphatic dicarboxylic acid which corresponds to the general formula $HOOC\text{-}(CH_2)_x\text{-}COOH$, where X is 2 to 10.

**25.** The film according to claim 24, **characterized in that** the nickel(II) dicarboxylic acid salt has an average particle size of less than 500 nm, wherein the average particle size is determined and scanning electron micrographs (SEM), as described under the heading "agglomerates and particle size" in the specification.

**26.** The film according to claim 24 or 25, **characterized in that** the nickel(II) dicarboxylic acid salt has an average particle size of more than 500 nm up to 5 $\mu$m, wherein the average particle size is determined on scanning electron micrographs (SEM) as described under the heading "agglomerates and particle size" in the specification.


**Revendications**

**1.** Procédé de préparation de polypropylène comportant une proportion de polypropylène $\beta$-cristallin, **caractérisé en ce que**

• l'on mélange au moins un sel d'acide dicarboxylique de nickel(II) et au moins un polypropylène, et
• ledit sel d'acide dicarboxylique de nickel(II) correspond à la formule générale $[Ni^{2+}(^-OOC\text{-}(CH_2)_x\text{-}COO^-)]$ et ledit acide dicarboxylique est un acide dicarboxylique aliphatique saturé correspondant à la formule générale $HOOC\text{-}(CH_2)_x\text{-}COOH$ dans laquelle X est compris entre 2 et 10, et
• l'on fait fondre le mélange à une température d'au moins 150 °C, et
• l'on procède ensuite à un refroidissement faisant en sorte que le polypropylène fondu présente, suite audit refroidissement, une proportion de polypropylène $\beta$-cristallin supérieure à 50 et inférieure ou égale à 100 % en poids, ladite proportion $\beta$-cristalline étant mesurée par calorimétrie différentielle à balayage comme dans la description sous le titre Teneur en $\beta$.

**2.** Procédé de préparation de polypropylène comportant une proportion de polypropylène $\beta$-cristallin, **caractérisé en ce que**

• l'on mélange au moins un sel d'acide dicarboxylique de nickel(II) et au moins un polypropylène, et
• ledit sel d'acide dicarboxylique de nickel(II) correspond à la formule générale $[Ni^{2+}(^-OOC\text{-}(CH_2)_x\text{-}COO^-)]$ et ledit acide dicarboxylique est un acide dicarboxylique aliphatique saturé correspondant à la formule générale $HOOC\text{-}(CH_2)_x\text{-}COOH$ dans laquelle X est compris entre 2 et 10, et
• l'on fait fondre le mélange à une température d'au moins 150 °C, et
• l'on procède ensuite à un refroidissement faisant en sorte que le polypropylène fondu présente, suite audit refroidissement, une proportion de polypropylène $\beta$-cristallin supérieure à 10 et inférieure ou égale à 50 % en poids, ladite proportion $\beta$-cristalline étant mesurée par calorimétrie différentielle à balayage comme dans la description sous le titre Teneur en $\beta$.

**3.** Procédé selon la revendication 1, **caractérisé en ce que** ledit refroidissement du polypropylène fondu permet d'obtenir une proportion de $\beta$ comprise entre 70 et < 100 %.

**4.** Procédé selon l'une des revendications 1 à 3, **caractérisé en ce que** ledit refroidissement du polypropylène fondu est réalisé dans une plage de températures allant de 100 à 140 °C.

**5.** Procédé selon l'une des revendications 1 à 4, **caractérisé en ce que** ledit polypropylène est un homopolymère de propylène et/ou un copolymère de propylène et/ou un terpolymère de propylène.

**6.** Procédé selon l'une des revendications 1 à 5, **caractérisé en ce que** ledit polypropylène est un polypropylène isotactique ayant un point de fusion compris entre 140 et 170 °C.

**7.** Procédé selon l'une des revendications 1 à 6, **caractérisé en ce que** ledit sel d'acide dicarboxylique de nickel(II) présente une taille moyenne des particules comprise entre 1 et 500 nm, ladite taille moyenne des particules étant déterminée à l'aide de clichés réalisés par microscopie électronique à balayage (MEB) comme dans la description sous le titre Agglomérés et Taille des particules.

**8.** Procédé selon l'une des revendications 1 à 6, **caractérisé en ce que** ledit sel d'acide dicarboxylique de nickel(II) présente une taille moyenne des particules supérieure à 500 nm et inférieure ou égale à 5 $\mu$m, ladite taille moyenne des particules étant déterminée à l'aide de clichés réalisés par microscopie électronique à balayage (MEB) comme dans la description sous le titre Agglomérés et Taille des particules.

**9.** Procédé selon l'une des revendications 1 à 8, **caractérisé en ce que** ledit sel d'acide dicarboxylique de nickel(II) est un adipate ou pimélate ou subérate.

**10.** Procédé selon l'une des revendications 1 à 9, **caractérisé en ce que** ledit sel d'acide dicarboxylique de nickel(II) est pourvu d'un revêtement de surface constitué d'acides gras à longue chaîne, préférentiellement acide oléique, acide stéarique, de silanes, d'amines ou de sulfonates.

**11.** Procédé selon l'une des revendications 1 à 10, **caractérisé en ce que** l'on prépare une formulation complète à partir de polypropylène et de sel d'acide dicarboxylique de nickel(II) pour ensuite mélanger cette formulation complète avec du polypropylène, le faire fondre puis le refroidir.

**12.** Procédé de fabrication d'une feuille plate biaxialement orientée, **caractérisé en ce que**

• l'on mélange du sel d'acide dicarboxylique de nickel(II) et du polypropylène, et
• l'on les fait fondre, dans une extrudeuse, à une température d'au moins 150 °C, et
• l'on procède à l'extrusion de la matière en fusion à travers une filière plate, et
• l'on soumet la matière en fusion à un refroidissement pour obtenir une pré-feuille de manière à ce que la proportion de polypropylène $\beta$-cristallin soit d'au moins 50 %, ladite proportion $\beta$-cristalline étant mesurée par calorimétrie différentielle à balayage comme il est décrit dans la description sous le titre Teneur en $\beta$, et
• l'on chauffe ensuite ladite pré-feuille pour l'orienter longitudinalement et transversalement de manière à ce que le polypropylène $\beta$-cristallin de la pré-feuille se transforme en modification alpha du polypropylène,
• lesdits sels d'acide dicarboxylique de nickel(II) correspondant à la formule générale [Ni$^{2+}$($^-$OOC-(CH$_2$)$_x$-COO$^-$)] et lesdits acides dicarboxyliques étant des acides dicarboxyliques aliphatiques saturés correspondant à la formule générale HOOC-(CH$_2$)$_x$-COOH dans laquelle X est compris entre 2 et 10.

**13.** Procédé selon la revendication 12, **caractérisé en ce que** la feuille biaxialement orientée est poreuse.

**14.** Mélange ou formulation complète à partir de polypropylène et de sel d'acide dicarboxylique de nickel(II), **caractérisé en ce que** ledit sel d'acide dicarboxylique de nickel(II) présente une taille moyenne des particules inférieure à 500 nm, ladite taille moyenne des particules étant déterminée à l'aide de clichés réalisés par microscopie électronique à balayage (MEB) comme dans la description sous le titre Agglomérés et Taille des particules, et sel d'acide dicarboxylique de nickel(II) correspond à la formule générale [Ni$^{2+}$($^-$OOC-(CH$_2$)$_x$-COO$^-$)] et lesdits acides dicarboxyliques sont des acides dicarboxyliques aliphatiques saturés correspondant à la formule générale HOOC-(CH$_2$)$_x$-COOH dans laquelle X est compris entre 2 et 10.

**15.** Mélange ou formulation complète selon la revendication 14, **caractérisé en ce que** ledit polypropylène est un homopolymère de propylène isotactique, un copolymère de propylène, un terpolymère de propylène ou un mélange constitué d'un ou de plusieurs desdits polypropylènes.

**16.** Mélange ou formulation complète selon les revendications 14 ou 15, **caractérisé en ce que** l'homopolymère de propylène isotactique présente une isotacticité d'au moins 95 %, ladite isotacticité étant mesurée en soumettant la partie de l'homopolymère de propylène isotactique, laquelle est insoluble dans du n-heptane, à une spectroscopie par $^{13}$C-RMN (triade).

**17.** Mélange ou formulation complète selon les revendications 14 ou 15, **caractérisé en ce que** l'homopolymère de propylène isotactique présente une isotacticité inférieure à 95 %, ladite isotacticité étant mesurée en soumettant la partie de l'homopolymère de propylène isotactique, laquelle est insoluble dans du n-heptane, à une spectroscopie par $^{13}$C-RMN (triade).

**18.** Mélange ou formulation complète selon l'une des revendications 14 à 17, **caractérisé en ce que** ledit mélange ou ladite formulation complète présente une proportion maximale de β qui est > 90 %, ladite proportion maximale de β étant déterminée à partir de la deuxième courbe de réchauffement d'une mesure par calorimétrie différentielle à balayage comme dans la description sous le titre Teneur en β.

**19.** Mélange ou formulation complète selon l'une des revendication 14 à 18, **caractérisé en ce que** le sel d'acide dicarboxylique de nickel(II) est un pimélate de Ni (II), un adipate de Ni (II) ou un subérate de Ni (II) .

**20.** Mélange ou formulation complète selon l'une des revendications 14 à 19, **caractérisé en ce que** le sel d'acide dicarboxylique de nickel(II) est revêtu d'acides gras à longue chaîne, de silanes, d'aminés ou de sulfonates, préférentiellement d'acide oléique ou d'acide stéarique.

**21.** Utilisation de sels d'acide dicarboxylique de nickel(II) en tant qu'agents de nucléation β, lesdits sels d'acide dicarboxylique de nickel(II) correspondant à la formule générale $[Ni^{2+}(^-OOC-(CH_2)_x-COO^-)]$ et lesdits acides dicarboxyliques étant des acides dicarboxyliques aliphatiques saturés correspondant à la formule générale $HOOC-(CH_2)_x-COOH$ dans laquelle X est compris entre 2 et 10.

**22.** Procédé de préparation de sels d'acide dicarboxylique de nickel(II) à l'échelle nano, consistant à préparer une solution aqueuse d'un acide dicarboxylique et à ajouter une solution de sel de Ni (II) ou un sel de Ni (II) à ladite solution aqueuse puis à isoler et sécher le sel d'acide dicarboxylique de nickel(II) précipité et à réaliser, à partir du sel d'acide dicarboxylique de nickel(II) séché, une suspension dans un milieu liquide non-aqueux puis traiter ladite suspension de manière à réduire la granulométrie des sels d'acide dicarboxylique de nickel(II) mis en suspension, lesdits sels d'acide dicarboxylique de nickel (II) correspondant à la formule générale $[Ni^{2+}(^-OOC-(CH_2)_x-COO^-)]$ et lesdits acides dicarboxyliques étant des acides dicarboxyliques aliphatiques saturés correspondant à la formule générale $HOOC-(CH_2)_x-COOH$ dans laquelle X est compris entre 2 et 10.

**23.** Procédé selon la revendication 22, **caractérisé en ce que** ledit sel de Ni (II) est du $NiCl_2$, du carbonate de nickel ou du $Ni(OH)_2$.

**24.** Feuille, comprenant du polypropylène et du sel d'acide dicarboxylique de nickel(II), ledit sel d'acide dicarboxylique de nickel(II) correspondant à la formule générale $[Ni^{2+}(^-OOC-(CH_2)_x-COO^-)]$ et ledit acide dicarboxylique étant un acide dicarboxylique aliphatique saturé correspondant à la formule générale $HOOC- (CH_2)_x-COOH$ dans laquelle X est compris entre 2 et 10.

**25.** Feuille selon la revendication 24, **caractérisée en ce que** ledit sel d'acide dicarboxylique de nickel(II) présente une taille moyenne des particules inférieure à 500 nm, ladite taille moyenne des particules étant déterminée à l'aide de clichés réalisés par microscopie électronique à balayage (MEB) comme dans la description sous le titre Agglomérés et Taille des particules.

**26.** Feuille selon les revendications 24 ou 25, **caractérisée en ce que** ledit sel d'acide dicarboxylique de nickel(II) présente une taille moyenne des particules supérieure à 500 nm et inférieure ou égale à 5 μm, ladite taille moyenne des particules étant déterminée à l'aide de clichés réalisés par microscopie électronique à balayage (MEB) comme dans la description sous le titre Agglomérés et Taille des particules.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1188278
- US 3540979 A
- DE 3610644
- DE 4420989
- EP 0557721 A

**In der Beschreibung aufgeführte Nicht-Patentliteratur**

- *Macromol. Mater. Eng.,* 2000, vol. 275, 8-17
- *Macromol. Rapid Commun,* 2001, vol. 22, 176-180